# EUROPEAN PATENT APPLICATION

(11) **EP 3 421 072 A1**
(43) Date of publication of application: **02.01.2019**
(21) Application number: 17756808.6
(22) Date of filing: 23.02.2017
(51) Int. Cl.: A61M 25/06, A61M 25/09

(54) **SAFETY CATHETER ASSEMBLY**

(30) Priority: 25.02.2016 KR 20160022409
(71) Applicant: Kim, Jung Gyu, Seoul 06090 (KR); Jang, Hong Sun, Seoul 06574 (KR)
(72) Inventor: Kim, Jung Gyu, Seoul 06090 (KR); Jang, Hong Sun, Seoul 06574 (KR)
(74) Representative: Körfer, Thomas
(86) International application number: PCT/KR2017/001962
(87) International publication number: WO 2017/146470

(57) **Abstract**

The present invention relates to a safety catheter assembly. One aspect of the present invention provides a safety catheter assembly which comprises: a catheter having a tube; and a needle assembly, wherein the needle assembly comprises: a housing which is detachably coupled to the catheter; a handle member which is provided to be movable forward and backward within the housing; a needle which is mounted on the handle member; a fixing member for fixing the handle member to the housing in a forwardly moved position of the handle member when the handle member is forwardly moved such that the needle is exposed to the outside by passing through the catheter; and an elastic member for backwardly moving the handle member such that the needle is inserted into the housing when the handle member is released from the fixed state.

## Description

### [Technical Field]

The present invention relates to a safety catheter assembly, and more particularly, to a safety catheter assembly which is capable of compressing a portion of a connector to prevent blood from flowing out until a fluid connection is established, and is configured such that the tube inserted into a blood vessel is not affected even if the fluid line is moved.

### [Background Art]

In general, vascular catheters, for example, intravenous catheters (IV catheters), are used to administer fluids, such as saline, various medications, and overall parenteral nutritional supplements, to a patient, or to draw blood from a patient.

A catheter assembly is intended to leave a connector having a tube with a subject, thereby providing convenience such that newly piercing the skin is not required when administering a medical fluid through injection or the like.

In order to insert the tube of the catheter assembly into the body of the subject, a separate guide needle is inserted into the body of the subject along with the tube, and then only the guide needle is withdrawn and removed.

In the process of removing the guide needle, the operator or the subject may be injured by the guide needle, and thus may be infected.

### [Disclosure]

### [Technical Problem]

It is an object of the present invention to provide a safety catheter assembly capable of eliminating the risk of injury and infection in the process of removing a needle after a catheter procedure.

It is an object of the present invention to provide a safety catheter assembly which facilitates insertion of a guide wire.

It is another object of the present invention to provide a safety catheter assembly which is capable of blocking blood from flowing out toward the needle assembly during the catheter procedure and is excellent in operability.

### [Technical Solution]

In accordance with one aspect of the present invention, provided is a safety catheter assembly comprising a catheter having a tube; and a needle assembly comprising a housing coupled to detachably coupled to the catheter, a handle member configured to advance and retreat within the housing, a needle mounted on the handle member, a fixing member for fixing the handle member to the housing at an advanced position when the handle member advances to allow the needle to be exposed to an outside through the catheter, and an elastic member for causing the handle member to retreat such that the needle is inserted into the housing when the handle member is released from a fixed state.

In accordance with another aspect of the present invention, provided is a safety catheter assembly comprising a catheter including a tube and a connector connected to the tube; and a needle assembly comprising a housing detachably coupled to the connector, a handle member configured to advance and retreat within the housing, a needle mounted on the handle member, a fixing member for fixing the handle member to the housing to maintain an advanced state of the handle member when the handle member advances to allow the needle to be exposed to an outside through the tube, and an elastic member for causing the handle member to retreat such that the needle is inserted into the housing when the handle member is released from a fixed state. Here, the connector comprises a first region connected to the tube, a second region extending from the first region and configured to be compressible, and a third region extending from the second region, the housing being mounted on the third region.

In accordance with yet another aspect of the present invention, provided is a safety catheter assembly comprising a catheter having a tube; and a needle assembly comprising a housing detachably coupled to the catheter, a handle member configured to advance and retreat within the housing, a needle mounted on the handle member, a fixing member for fixing the handle member to the housing at an advanced position when the handle member advances to allow the needle to be exposed to an outside through the catheter, and an elastic member for causing the handle member to retreat such that the needle is inserted into the housing when the handle member is released from a fixed state. Here, the handle member includes a space connected to the needle to allow movement of a fluid therethrough. In addition, the space is open to the outside such that the guide wire is inserted into the handle member from the outside.

In accordance with still yet another aspect of the present invention, provided is a safety catheter assembly comprising a catheter having a tube and a connector connected to the tube; and a needle assembly coupled to the catheter.

Here, the needle assembly comprises a housing coupled to the connector, a handle member configured to advance and retreat within the housing, a needle mounted on the handle member, a fixing member for fixing the handle member to the housing at an advanced position when the handle member advances to allow the needle to be exposed to an outside through the catheter, and an elastic member for causing the handle member to retreat such that the needle is inserted into the housing when the handle member is released from a fixed state.

In addition, the connector comprises a first region connected to the tube, a second region extending from the first region and configured to be compressible, and a third region extending from the second region, the housing being mounted on the third region.

Further, the first and third regions have greater stiffness than the second region.

The housing may be provided with a first fixing hole at the position to which the handle member advances, wherein the fixing member may be arranged to be inserted into the first fixing hole.

In addition, the fixing member may be configured to be compressible in a diameter direction of the housing.

Further, the elastic member may comprise a spring configured to be pulled as the handle member advances.

The handle member may have a space connected to the needle, wherein the space may be arranged to be open to the outside of the handle member.

The space is connected to an interior of the needle to allow movement of a fluid therethrough.

The housing may be fixed to the catheter by rotating in a first circumferential direction and be separated from the catheter by rotating in a second circumferential direction opposite to the first circumferential direction.

An open end portion positioned outside the housing and connected to the space may have at least one part having a cross-sectional area that narrows as the open end portion extends towards the needle.

A diameter of the open end portion may be greater than a diameter of the space.

In addition, an inner circumferential surface of the open end portion may comprise a curved surface.

A leading end region of a space adjacent to the needle may be configured to have a cross-sectional area that narrows as the leading end region extends toward the needle.

Further, the leading end region may comprise an inclined surface.

The second region may be formed of a compressible material.

The first and third regions may be formed of a resin material.

The second region may be formed of a silicone material.

The housing may be detachably coupled to the connector.

### [Advantageous Effects]

As is apparent from the above, a safety catheter assembly according to at least one embodiment of the present invention has the following effects.

The needle assembly can be separated from the catheter by causing the needle to retreat into the needle assembly after the catheter procedure so as not to be exposed to the outside. Therefore, the risk of injury and infection caused by the needle in the process of separating the needle assembly may be eliminated.

In addition, insertion of the guide wire may be facilitated due to the needle assembly.

In the catheter procedure, flow of blood to the needle assembly can be easily interrupted. Specifically, since a region of the connector is compressible, blood can be prevented from flowing out until a fluid connection is established. Further, even if the fluid line is moved, the tube inserted into the blood vessel is not affected.

### [Description of Drawings]

FIG. 1 is a view illustrating a safety catheter assembly related to an embodiment of the present invention, wherein a catheter and a needle assembly are separated from each other.
FIG. 2 is a view illustrating coupling between the catheter and the needle assembly of FIG. 1.
FIG. 3 is a view illustrating an exemplary usage of the safety catheter assembly of FIG. 2.
FIG. 4 is an enlarged view illustrating the catheter of FIG. 1.
FIG. 5 is a view illustrating the assembly structure of the catheter and the needle assembly of FIG. 1.
FIG. 6 is a view illustrating another usage of the safety catheter assembly related to an embodiment of the present invention.
FIG. 7 is a view illustrating the safety catheter assembly of FIG. 6 and a guide wire.
FIG. 8 is an enlarged view of a region of the needle assembly of FIG. 1.

### [Best Mode]

Hereinafter, a safety catheter assembly according to an embodiment of the present invention will be described in detail with reference to the accompanying drawings.

The same or similar reference numerals are assigned to the same or corresponding elements throughout the drawings, and redundant description thereof will be omitted. For simplicity, the size and shape of each constituent member shown in the drawings can be exaggerated or reduced.

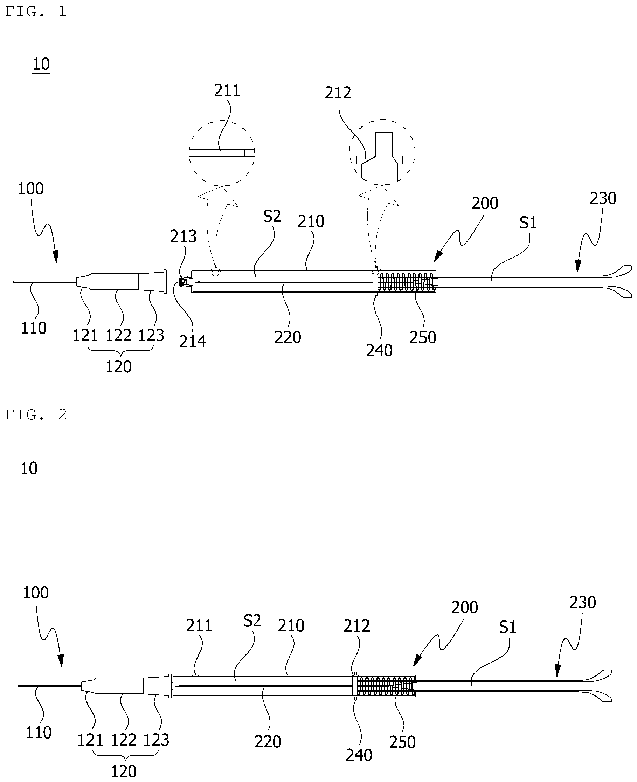

FIG. 1 is a view illustrating a safety catheter assembly 10 related to an embodiment of the present invention, wherein a catheter 100 and a needle assembly 200 are separated from each other, and FIG. 2 is a view illustrating coupling between the catheter 100 and the needle assembly 200 of FIG. 1.

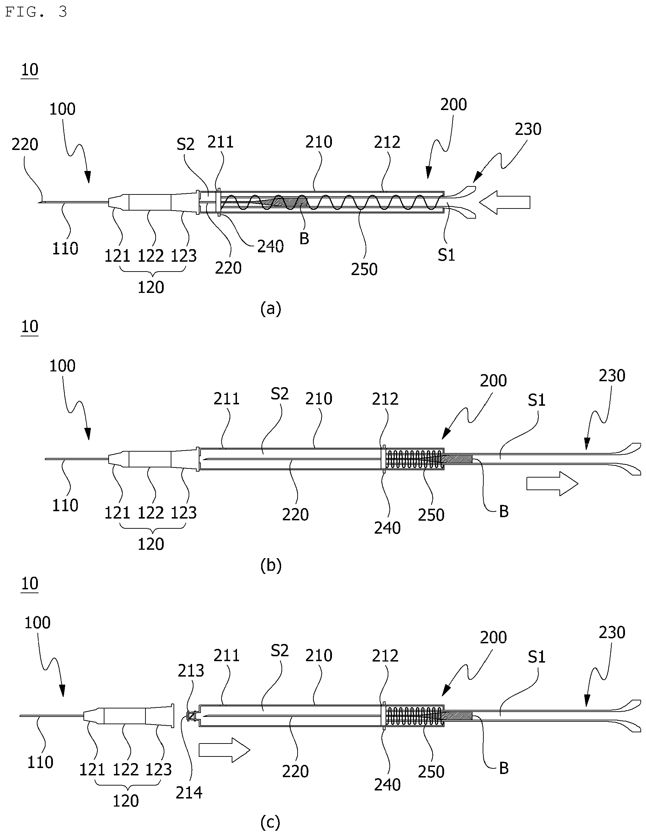

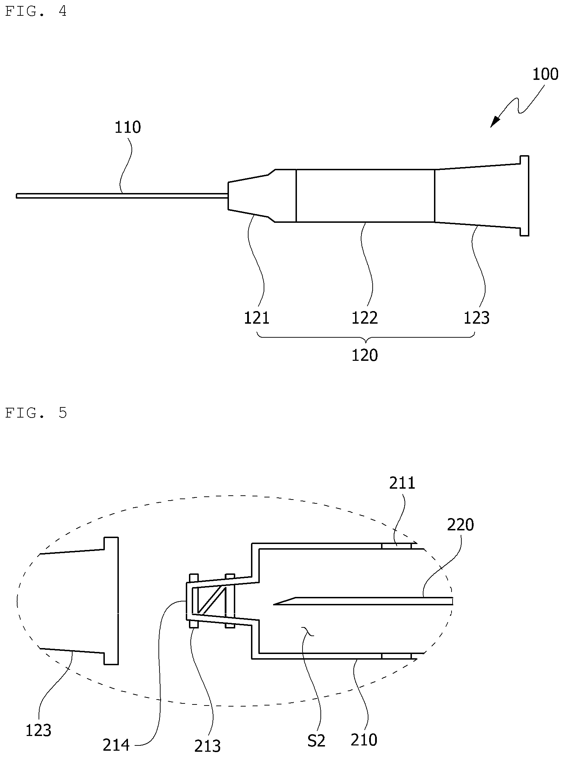

FIG. 3 is a view illustrating an exemplary usage of the safety catheter assembly 10 of FIG. 2, FIG. 4 is an enlarged view illustrating the catheter 100 of FIG. 1, and FIG. 5 is a view illustrating the assembly structure of the catheter 100 and the needle assembly 200 of FIG. 1.

Referring to FIGS. 1 and 2, a safety catheter assembly 10 according to an embodiment of the present invention includes a catheter 100 and a needle assembly 200, which is coupled to the catheter 100. The needle assembly 200 may be detachably coupled to the catheter 100.

Referring to FIGS. 1 and 4, the catheter 100 includes a tube 110 to be inserted into a subject.

The catheter 100 also includes the tube 110 and a connector 120 connected to the tube 110.

The connector 120 may be configured to have a greater diameter than the tube 110. The connector 120 is connected to the tube 110 and a housing 210 of the needle assembly 200 is fixed to the connector 120.

Referring to FIG. 4, at least two regions of the connector 120 may be formed of different materials. For example, the connector 120 includes a first region 121 connected to the tube 110, a second region 122 extending from the first region 121 and configured to be compressible, and a third region 123 extending from the second region 122 and having the housing 210 mounted thereon. The operator can apply pressure to and compress the second region 122, thereby easily blocking blood B from flowing toward the needle assembly 200 during the procedure of the catheter 100.

Further, in order to improve operability, the first and third regions 121 and 123 may be configured to have greater stiffness than the second region 122. Specifically, the second region 122 may be formed of silicone. In addition, the first and third regions 121 and 123 may be formed of the same resin material, for example, polycarbonate.

The needle assembly 200 includes the housing 210, a handle member 230, a needle 220, a fixing member 240, and an elastic member 240.

Specifically, the needle assembly 200 includes the housing 210 detachably coupled to the catheter 100 (specifically, the connector), and the handle member 230 configured to advance or retreat within the housing 210.

In this specification, "advance" of the handle member 230 means that the handle member 230 is moved toward the catheter 100 with the catheter 100 and the needle assembly 200 coupled to each other. "Retreat" of the handle member 230 means that the handle member 230 is moved away from the catheter 100. The handle member 230 is a part with which an operator operates the needle assembly 200, and a region thereof is inserted into the housing 210, and the other region is exposed to the outside of the housing 210. In addition, at least one region of the handle member 230 is exposed to the outside of the housing 210 for the procedure even when the handle member 230 advances.

The needle assembly 200 includes the needle 220 mounted on the handle member 230, and the fixing member 240 configured to fix the handle member 230 to the housing 210 at an advanced position when the handle member 230 is caused to advance (see FIG. 3(a)) such that the needle 220 is exposed to the outside of the catheter 100, specifically, through the tube 110.

The needle assembly 200 includes an elastic member 250 configured to cause the handle member 230 to retreat such that the needle 220 is inserted into the housing 210 when the handle member 230 is released from the fixed state.

The housing 210 may have a cylindrical shape and has a first through hole 213 formed at a front end portion thereof (a region adjacent to the connector) and allowing the needle 220 to pass therethrough. The housing 210 also has a space S2 (also referred to as a "housing interior") in which the needle 220, the elastic member 250, and the fixing member 240 can be disposed, respectively. In addition, the housing 210 has a second through hole formed at a rear end portion thereof (on the opposite side of the front end) into which the handle member 230 is movably inserted.

In addition, a fixing portion 214 for coupling with the connector 120 may be provided at the front end portion of the housing 210. The fixing portion 214 may be embodied as a protruding portion having a predetermined shape so as to be selectively engaged. For example, the fixing portion 214 may have a Luer structure. Through the engagement structure of the fixing portion 214, the housing 210 may be fixed to the catheter 100 (specifically, the connector) by rotating in a first circumferential direction (for example, the clockwise direction) and be separated from the catheter 100 (specifically, the connector) by rotating in a second circumferential direction (e.g., the counterclockwise direction) opposite to the first circumferential direction.

The housing 210 is provided with a first fixing hole 211 at a position to the handle member 230 advances. In addition, the fixing member 240 may be arranged to be inserted into the first fixing hole 211. For this purpose, the fixing member 240 may be configured to be compressible in the diameter direction of the housing 210. In addition, a second fixing hole 212 is formed in the housing 210 at a position to which the handle member 230 retreats. The fixing member 240 may be arranged to be inserted into the second fixing hole 212. The fixing member 240 may be fixed to the handle member 230, and thus the fixing member 240 is moved according to advance and retreat of the handle member 230.

The first fixing hole 211 and the second fixing hole 212 may be arranged at a predetermined distance from each other in the housing 210. For example, the first fixing hole 211 may be provided at the front end of the housing 210, and the second fixing hole 212 may be provided at the rear end of the housing 210. The first fixing hole 211 should be formed at a position where the first fixing hole allows the needle 220 to be exposed to the outside of the tube 110 through the catheter 100 when the fixing member 240 is inserted into the first fixing hole 211 and the advanced position of the handle member 230 is fixed. Similarly, the second fixing hole 212 should be formed at a position to which the needle 220 retreats into the inner space S2 of the housing 210 so as not to be exposed to the outside of the housing 210 when the fixing member 240 is inserted into the second fixing hole 212 and the retreated position of the handle member 230 is fixed.

The elastic member 250 may include a spring configured to be pulled as the handle member 230 advances. One longitudinal end portion of the elastic member 250 may be fixed to the housing 210 and the other longitudinal end portion of the elastic member 250 may be fixed to the fixing member 240. In particular, the elastic member 250 may be arranged to surround at least a part of the region of the handle member 230. Further, the elastic member 250 may be disposed at the rear end portion of the housing 210. Specifically, the elastic member 250 is disposed in a space between the fixing member 240 and the second through hole. In this structure, when the handle member 230 advances, the elastic member 250 is pulled according to movement of the fixing member 240.

Referring to FIG. 3, in the catheter procedure, when the operator advances the handle member 230, the elastic member 250 is pulled and the fixing member 240 advances. Then, when the needle 220 is exposed to the outside of the catheter 100, the fixing member 240 is inserted into the first fixing hole 211, and the advanced position of the handle member 230 is maintained. If the operator pushes the fixing member 240 inserted into the first fixing hole 211 after inserting the catheter 100 into the subject, the fixing member 240 is separated from the first fixing hole 211, and is forced to retreat together with the handle member by the restoring force of the elastic member 250. Thereafter, when the needle 220 is completely inserted into the housing 210 without being exposed to the outside, the fixing member 240 is inserted into the second fixing hole 212, and the retreated position of the handle member 230 is maintained. As described above, the fixing member 240 is configured to be compressible in the radial direction of the housing 210. For example, the fixing member 240 may be in a compressed state in the radial direction of the housing 210 when the fixing member 240 moves (advances and retreats) within the housing 210. As the fixing member 240 is released from the compressed state in the first and second fixing holes 211 and 212, the fixing member 240 may be inserted into the first and second fixing holes 211 and 212.

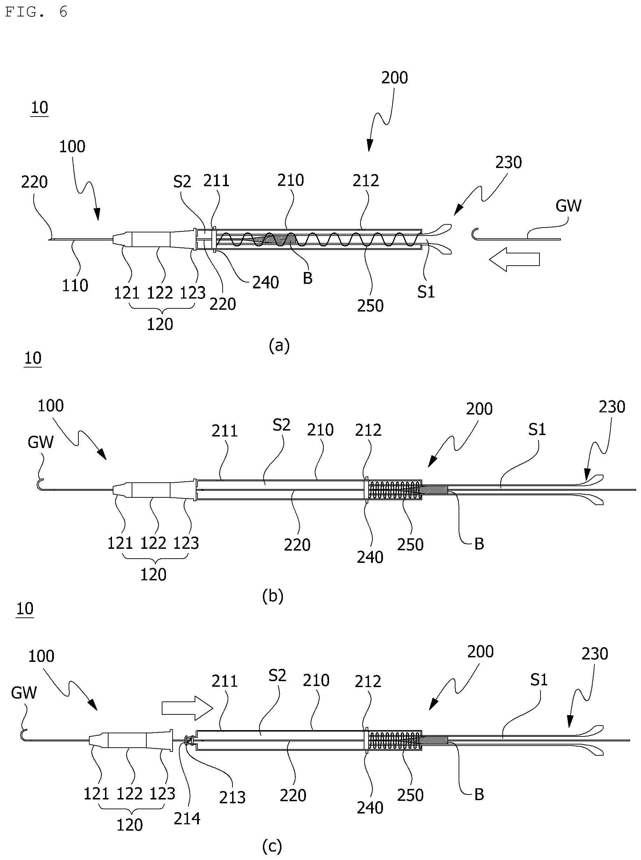

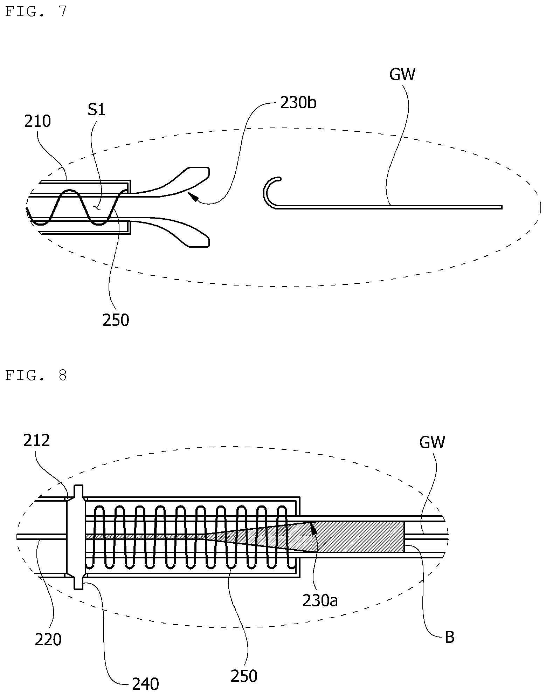

FIG. 6 is a view illustrating another usage of the safety catheter assembly 10 related to an embodiment of the present invention, FIG. 7 is a view illustrating the safety catheter assembly 10 of FIG. 6 and a guide wire GW, and FIG. 8 is an enlarged view of a region of the needle assembly 200 of FIG. 1.

The handle member 230 includes a space S1 connected to the needle 220 to allow movement of a fluid therethrough. The space S1 is arranged to be open to the outside of the handle member.

Referring to FIGS. 6 and 7, the space S1 is open to the outside such that the guide wire GW can be inserted into the handle member 230 from the outside. An open end 230b, which is positioned outside the housing 210 and is connected to the space S1, is configured such that at least a part thereof has a cross-sectional area which is narrowed as the open end portion extends toward the needle 220. In addition, the diameter of the open end 230b may be larger than the diameter of the space S1. Specifically, the open end 230b of the handle member, which is connected to the space, may be provided with at least one part configured such that the cross-sectional area thereof becomes narrower toward the needle 210 so as to guide insertion of the guide wire. For example, the inner circumferential surface of the open end 230b may include an inclined surface or a curved surface.

Referring to FIG. 8, a leading end region 230a of the space S1 of the handle member 230 adjacent to the needle 220 is formed to have a cross-sectional area which is narrowed toward the needle 220. The leading end region 230a of the space S1 adjacent to the needle 220 may be formed to have a cross-sectional area which is narrowed toward the needle 220 in order to guide the guide wire GW into the needle. In addition, the leading end region 230a may include an inclined surface.

Although some embodiments of the present invention have been disclosed for illustrative purposes, it is to be understood that the invention is not limited to the disclosed exemplary embodiments. Those skilled in the art will appreciate that various modifications, variations and additions can be made to the present invention, without departing from the scope and spirit of the invention as disclosed in the accompanying claims.

### [industrial Applicability]

According to the present invention, the needle assembly can be separated from the catheter by causing the needle to retreat into the needle assembly after the catheter procedure so as not to be exposed to the outside. Therefore, the risk of injury and infection caused by the needle in the process of separating the needle assembly may be eliminated.

## Claims

1. A safety catheter assembly comprising:
a catheter having a tube and a connector connected to the tube; and
a needle assembly coupled to the catheter,
wherein the needle assembly comprises:
a housing coupled to the connector;
a handle member configured to advance and retreat within the housing;
a needle mounted on the handle member;
a fixing member for fixing the handle member to the housing at an advanced position when the handle member advances to allow the needle to be exposed to an outside through the catheter; and
an elastic member for causing the handle member to retreat such that the needle is inserted into the housing when the handle member is released from a fixed state,
wherein the connector comprises:
a first region connected to the tube;
a second region extending from the first region and configured to be compressible; and
a third region extending from the second region, the housing being mounted on the third region,
wherein the first and third regions have greater stiffness than the second region.

2. The safety catheter assembly according to claim 1,
wherein the housing is provided with a first fixing hole at the position to which the handle member advances,
wherein the fixing member is arranged to be inserted into the first fixing hole.

3. The safety catheter assembly according to claim 2,
wherein the fixing member is configured to be compressible in a diameter direction of the housing.

4. The safety catheter assembly according to claim 3,
wherein the elastic member comprises a spring configured to be pulled as the handle member advances.

5. The safety catheter assembly according to claim 1,
wherein the handle member has a space connected to the needle,
wherein the space is arranged to be open to the outside of the handle member.

6. The safety catheter assembly according to claim 1,
wherein the housing is fixed to the catheter by rotating in a first circumferential direction and is separated from the catheter by rotating in a second circumferential direction opposite to the first circumferential direction.

7. The safety catheter assembly according to claim 1,
wherein an open end portion positioned outside the housing and connected to the space has at least one part having a cross-sectional area that narrows as the open end portion extends towards the needle.

8. The safety catheter assembly according to claim 7,
wherein a diameter of the open end portion is greater than a diameter of the space.

9. The safety catheter assembly according to claim 7,
wherein an inner circumferential surface of the open end portion comprises a curved surface.

10. The safety catheter assembly according to claim 1,
wherein a leading end region of a space adjacent to the needle is configured to have a cross-sectional area that narrows as the leading end region extends toward the needle.

11. The safety catheter assembly according to claim 10,
wherein the leading end region comprises an inclined surface.

12. The safety catheter assembly according to claim 1,
wherein the second region is formed of a compressible material.

13. The safety catheter assembly according to claim 1,
wherein the first and third regions are formed of a resin material.

14. The safety catheter assembly according to claim 1,
wherein the second region is formed of a silicone material.

15. The safety catheter assembly according to claim 1,
wherein the housing is detachably coupled to the connector.
